# EUROPEAN PATENT SPECIFICATION

(11) **EP 0 428 979 B1**
(45) Date of publication and mention of the grant of the patent: **12.04.1995**
(21) Application number: 90121679.6
(22) Date of filing: 13.11.1990
(51) Int. Cl.: C07C 57/03, C07C 51/12

(54) **Carbonylation of allylic butenols and butenol esters**
Carbonylierung von Allylbutenolen und -butenolestern
Procédé de carbonylation des butènols allyliques et des esters butènoliques

(30) Priority: 13.11.1989 US 436360
(43) Date of publication of application: 29.05.1991
(73) Proprietor: E.I. DU PONT DE NEMOURS AND COMPANY, Wilmington Delaware 19898 (US)
(72) Inventor: Burke, Patrick Michael, Wilmington, Delaware 19803 (US)
(74) Representative: Abitz, Walter, Dr.-Ing.

(56) References cited:
- EP-A- 0 181 003
- FR-A- 2 180 021
- GB-A- 1 233 121

## Description

This invention relates to a process for the carbonylation of allylic butenols and butenol esters.

The use of rhodium catalysts has been disclosed for the carbonylation of a variety of saturated alcoholic compounds, including: saturated aliphatic alcohols, or the esters of such alcohols (U.S. 3,689,533, U.S. 3,717,670, U.S. 3,772,380, and U.S. 4,433,166); saturated diols or polyols, or the ester or cyclic ether derivatives of such diols (G.B. 1,278,353, G.B. 1,278,354, U.S. 3,689,533, U.S. 3,772,380, U.S. 3,813,428, and U.S. 4,060,547); and aromatic alcohols (U.S. 3,689,533 and U.S. 3,717,670).

Rhodium has also been used as catalyst in the carbonylation of unsaturated alcoholic compounds.

In U.S. 3,427,344, Tsuji et al. disclose the manufacture of unsaturated carboxylic acid esters by the reaction of allylic compounds with carbon monoxide and a cyclic ether in the presence of a Group VII or Group VIII noble metal catalyst. In all of the Examples of this reference, the allylic compound is an allylic halide.

Schreck (EP 144,936 A3) discloses a process for the production of organic carboxylic acids by the catalytic reaction of an alcohol and carbon monoxide in contact with an organic ester or a compound which under the reaction conditions is convertible to an organic ester and a homogeneous catalyst system containing rhodium metal atom and a mixture of lithium iodide and methyl iodide.

In U.S. 3,579,552, Craddock et al. disclose the use of an HI-promoted rhodium catalyst for the carbonylation of allyl alcohol to give a mixture of saturated monocarboxylic acids, n-butyric and isobutyric acids. The carbonylation of allylic butenols or butenol esters is not disclosed.

In U.S. 4,140,865, Fernholz et al. disclose the use of methyl iodide-promoted rhodium catalysts for the carbonylation of allyl alcohol, diallyl ether and allyl esters to give vinyl acetic acid and, optionally, γ-butyrolactone. The carbonylation of allylic butenols or butenol esters is not disclosed.

It has now been found that when a rhodium catalyst is promoted with low concentrations of HI or HBr, allylic butenols and butenol esters can be selectively carbonylated to 3-pentenoic acid under very mild conditions.

This invention is a process for the carbonylation of an allylic compound selected from the class consisting of butenols and butenol esters of monocarboxylic acids as defined below to form 3-pentenoic acid or a carboxylic acid anhydride thereof, wherein the process comprises reacting said allylic compound and carbon monoxide with a rhodium catalyst and a promoter selected from the group of hydrogen bromide and hydrogen iodide, at a temperature in the range of 10°C to 250°C and at a carbon monoxide partial pressure in the range of 1.7 to 206.9 bar gauge (25 to 3000 psig), wherein the concentration of catalyst is in the range of 0.005% to 0.50% by weight of the total weight of the reaction mixture, and the molar ratio of promoter to rhodium is between 1:1 and 10:1.

The process of this invention can be run either as a batch or as a continuous process.

This process is useful for the preparation of 3-pentenoic acid and carboxylic acid anhydride derivatives thereof. These products are useful as difunctional monomers and as intermediates in the synthesis of adipic acid.

Suitable allylic compounds are of the form 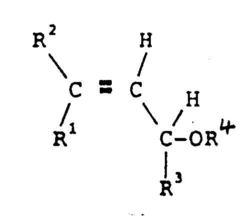
where
one of the groups R¹, R² and R³ is methyl and the other two groups are H; and
R⁴ is selected from the group consisting of H and 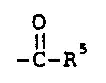
wherein R⁵ is selected from the group consisting of C₁ to C₁₀ alkyl.

The allylic butenols, 3-buten-2-ol and 2-buten-1-ol, are commercially available. Esters of allylic butenols 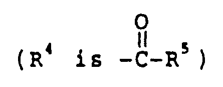
can be made by standard routes, such as the hydration of butadiene in a carboxylic acid solvent, by the acid catalyzed reaction of an allylic butenol with a suitable carboxylic acid, or by the reaction of an allylic butenyl halide with a carboxylic acid.

The temperature of the reaction is in the range of 10°C to 250°C. Below 10°C, the reaction becomes too slow to be commercially feasible, and above 250°C the formation of undesirable product leads to significant yield losses.

Suitable total pressures are in the range 1.7-206.9 bar gauge (25-3000 psig), with 6.90-137.9 bar gauge (100-2000 psig) being preferred and 13.8-69.0 bar gauge (200-1000 psig) being most preferred. The partial pressure of carbon monoxide is usually maintained in the range 1.7-206.9 bar gauge (25-3000 psig), preferably 6.90-69.0 bar gauge (100-1000 psig).

The source of the carbon monoxide (CO) for the present process is not particularly critical and commercially available grades of CO are satisfactory. The carbon monoxide can contain inert impurities such as carbon dioxide, methane, nitrogen, noble gases, and paraffinic hydrocarbons having from 1 to 4 carbon atoms. The carbon monoxide can also contain hydrogen.

Suitable solvents for this process are those which are compatible with the reactants and the catalyst system under the reaction conditions. Such solvents include aliphatic and aromatic carboxylic acid solvents, aromatic hydrocarbon solvents, saturated halocarbon solvents, and mixtures thereof. More specifically, suitable carboxylic acids include aliphatic C₂-C₂₀ monocarboxylic acids, aliphatic C₄-C₂₀ dicarboxylic acids, benzoic acid, C₇-C₁₀ alkyl-substituted benzoic acids, and mixtures thereof. Suitable aromatic hydrocarbon solvents include benzene and C₁-C₄ alkyl substituted benzenes. Suitable saturated halocarbon solvents include chlorinated hydrocarbons such as methylene chloride, dichloroethane and chloroform. The preferred solvents are methylene chloride, benzene, toluene, aliphatic C₂-C₆ monocarboxylic acids, C₄-C₇ dicarboxylic acids, benzoic acid and mixtures thereof. The most preferred solvents are acetic, propionic, butyric, 2-methylbutyric, valeric, and caproic acids, benzene, toluene, and mixtures thereof. Mixtures of monocarboxylic and dicarboxylic acids can also be used in whole or in part as the solvent for this process. Such monocarboxylic and dicarboxylic acids include adipic, valeric, 2-methylglutaric, ethylsuccinic and methylbutyric acids. Alternatively, the process of this invention may be run in the absence of solvent.

The rhodium catalyst can be provided from any source or by any material which will produce rhodium ions under hydrocarboxylation conditions. Among the materials which can be employed as the source of the rhodium catalyst are rhodium metal, rhodium salts, rhodium oxides, rhodium carbonyl compounds, organorhodium compounds, coordination compounds of rhodium, and mixtures thereof. Specific examples of such materials include, but are not limited to, rhodium(III)chloride and its hydrates, RhI₃, Rh(CO)₂I₃, Rh(CO)I₃, rhodium(III) nitrate trihydrate, Rh₄(CO)₁₂, Rh₆(CO)₁₆, Rh(acac)₃, Rh(CO)₂ (acac), Rh(C₂H₄)₂(acac), [Rh(C₂H₄)₂Cl]₂, [Rh(CO)₂Cl]₂, [Rh(CO)₂Br]₂, Rh(COD)(acac), [Rh(COD)Cl]₂, RhCl(CO)(PPh₃)₂, Rh₂[O₂C(CH₂)₆CH₃]₄ and Rh₂(acetate)₄, where acac is acetylacetonate and COD is 1,5-cyclooctadiene. Supported rhodium compounds, e.g. Rh/C and Rh/alumina, can also be used as a source of the rhodium catalyst. Rhodium compounds containing bidentate phosphine or nitrogen ligands should be avoided. Preferred sources of the rhodium catalyst include rhodium(I) compounds such as [Rh(CO)₂Cl]₂, [Rh(COD)Cl]₂, and Rh(COD)(acac) and rhodium iodide compounds such as RhI₃ and Rh(CO)₂I₃.

Suitable concentrations of rhodium in the reaction medium are in the range of 0.005-0.50% by weight of rhodium metal based on the weight of the reaction medium. Preferably, the concentration of rhodium is in the range of 0.01-0.20 wt%, more preferably 0.02-0.10 wt% Although high reaction rates on a per Rh basis can be obtained even at low concentrations of Rh, it is generall more economical to operate at Rh concentrations above 0.01 wt%. Similarly, Rh concentrations below 0.20 wt% are preferred to minimize the formation of unwanted by-products.

The rhodium catalyst, which can be preformed or formed in situ, must be promoted by HI or HBr, preferably HI, to achieve the high reaction rates and high selectivity to 3-pentenoic acid.

The molar ratio of promoter-to-rhodium can be in the range of 1:1 to 10:1. Although high selectivities to 3-pentenoic acid can be obtained even at low promoter-to-Rh ratios, the rate of formation of 3-pentenolc acid on a per Rh basis decreases significantly when the molar ratio of promoter-to-Rh is less than about 1. This decrease in reaction rates, coupled with the high cost of rhodium makes it more economical to use promoter-to-Rh ratios greater than 1:1. Similarly, the molar ratio of promoter-to-Rh must be less than about 10 to obtain reasonable yields of the unsaturated acid, rather than the generally less desirable saturated products, e.g., valeric acid. Preferably, the molar ratio of promoter-to-Rh is between 2:1 and 6:1.

The reaction of crotyl in the presence of water is preferred, wherein the carbon monoxide partial pressure is in the range of 6.90 to 69.0 bar gauge, the rhodium concentration is in the range of 0.01-0.20% based on the total weight of the reaction mixture, and the molar ratio of promotor to rhodium is between 2:1 and 6:1.

Reaction times can be varied and depend on the temperature, pressure, promoter and solvent. Residence times of 1 minute to 20 hours are acceptable.

Products can be isolated by standard techniques, e.g., by extraction or distillation, depending on the reaction mixture.

The type of allylic compound (alcohol or ester), the solvent and the amount of water used in the process of this invention significantly influence the types of products obtained. Carbonylation of allylic alcohols by the process of this invention gives high yields of 3-pentenoic acid in the absence of substantial amounts of water. In the presence of water, some conversion of 3-pentenoic acid to saturated dicarboxylic acids (adipic, methylglutaric and ethylsuccinic acids) is observed, especially at high temperatures. In the strict absence of water, and in non-carboxylic acid solvents, the esters of allylic butenols will be carbonylated to olefinically unsaturated carboxylic acid anhydrides. In the presence of small amounts of water or in the presence of carboxylic acids, these anhydrides may be converted to 3-pentenoic acid. In the presence of larger amounts of water, increasing amounts of saturated dicarboxylic acids are observed. Therefore, to maximize the formation of the desired 3-pentenoic acid, the process of this invention is carried out in the absence of substantial amounts of water when the substrate is an allylic butenol, but in the presence of small amounts of water (about 1-2 moles of water per mole of allylic compound) or in carboxylic acid solvent when the compound is an allylic ester.

The water used in the process of this invention can be obtained from water added to the reaction mixture or contained in the reagents (e.g., aqueous HI or hydrated metal salts) or solvents; it can also be formed under the reaction conditions (e.g., by the formation of anhydrides or esters). When the compound is an allylic ester, the molar ratio of water/compound in the reaction mixture is preferably less than about 5, more preferably less than about 2, most preferably less than about 1.

The type of product obtained by the process of this invention is also influenced by a number of other factors. Low temperatures, low partial pressures of CO, less polar solvents, HBr promoter, low promoter-to-rhodium ratios and short reaction times tend to favor the formation of 3-pentenoic acid. Conversely, high temperatures, high partial pressures of CO, more polar solvents, HI promoter and long reaction times tend to favor the formation of dicarboxylic acids. High promoter-to-rhodium ratios tend to favor the formation of saturated monocarboxylic acids, especially in carboxylic acid solvents at high temperatures.

As illustrated in the Examples below, the 3-pentenoic acid produced by the process of this invention is not always derived from CO insertion into the allylic C-O bond of the allylic compound. For example, carbonylation of 3-buten-2-ol gives predominantly cis- and trans-3-pentenoic acids, not 2-methyl-3-butenoic acid, as would be expected from direct insertion of CO into the allylic C-O bond.

The process of the invention is capable of carbonylating allylic butenols to 3-pentenoic acid at low temperatures and pressures (20-38°C, 6.90 bar (100 psi); Ex. 10, infra). Moreover, the selectivity to 3-pentenoic acid at these low temperature and pressure conditions is virtually quantitative.

The following examples are presented to illustrate, but not to restrict the present invention. Parts and percentages are by weight and temperatures are in degrees Celsius unless otherwise noted.

### Examples

### Example 1. Carbonylation of crotyl alcohol (2-buten-1-ol) at 60°C and 6.90 bar (100 psi).

A 300 mL glass-lined Hastelloy-C shaker tube was charged with glacial acetic (46 mL), crotyl alcohol (2.9 g, 40 mmoles), 57% aq HI (0.6 g, 2.7 mmole HI), water (1.4 g, 80 mmoles) and RhCl₃.3H₂O (0.21 g, 0.8 mmole). The tube was closed, cooled to -78°C, evacuated and then pressured with carbon monoxide to 6.90 bar (100 psi). The tube was agitated and heated to 60°C over a period of about 45 min. The total pressure at 60°C was about 8.96 bar (130 psi). The temperature was maintained at 60°C an additional 2 h and CO was added at 15 min intervals to maintain the total pressure constant at about 8.96-9.65 bar (130-140 psi). The reaction was terminated after 2 h by cooling to 0°C. The excess CO was slowly vented, the product was discharged and the tube was rinsed with methanol (2 x 20 mL).

The product and washings were combined and the solution was filtered. Internal standard (2.00 g o-dichlorobenzene) was added and the solution was made up to 200 mL with methanol. The sample was esterified with BF₃/methanol and then analyzed as the methyl esters.

Analysis showed the following yields (moles product formed per 100 moles of crotyl alcohol charged):

| | |
|---|---|
| 3-pentenoic acid (cis and trans) | 48.8 |
| trans-2-pentenoic acid | 1.3 |
| 2-methylbutyric acid | 0.0 |
| valeric acid | 0.0 |
| γ-valerolactone | 0.0 |
| adipic acid | 0.1 |
| 2-methylglutaric acid | 0.9 |
| ethylsuccinic acid | 0.0 |

In this and the following Examples, residual crotyl alcohol was not analyzed. This Example illustrates the remarkably high selectivity (98%) to monocarboxylic acids under mild carbonylation conditions.

### Example 2. Carbonylation of crotyl alcohol at 110°C and 34.5 bar (500 psi).

The procedure described in Example 1 was repeated except that the temperature was increased to 110°C, the pressure was increased to 34.5 bar (500 psi), and the 80 mmole of additional water was omitted (only the 17 mmole of water from the aq HI and RhCl₃.3H₂O was added). Analysis gave the following results:

| | |
|---|---|
| 3-pentenoic acid (cis and trans) | 2.7 |
| trans-2-pentenoic acid | 1.0 |
| 2-methylbutyric acid | 0.9 |
| valeric acid | 2.0 |
| γ-valerolactone | 10.0 |
| adipic acid | 23.0 |
| 2-methylglutaric acid | 36.2 |
| ethylsuccinic acid | 6.7 |

### Example 3. Carbonylation of crotyl alcohol at 180°C and 20.7 bar (300 psi).

The procedure described in Example 1 was repeated, except that the temperature was increased to 180°C and the pressure was increased to 20.7 bar (300 psi). Analysis gave the following results:

| | |
|---|---|
| 3-pentenoic acid (cis and trans) | 1.0 |
| trans-2-pentenoic acid | 0.3 |
| 2-methylbutyric acid | 4.9 |
| valeric acid | 10.5 |
| γ-valerolactone | 4.3 |
| adipic acid | 30.4 |
| 2-methylglutaric acid | 37.1 |
| ethylsuccinic acid | 8.1 |

The high yields of dicarboxylic acids illustrate that the first-formed monocarboxylic acids are further hydrocarboxylated under these reaction conditions.

### Example 4. Carbonylation of crotyl alcohol using a bromide promoter at 80°C and 34.5 bar (500 psi).

The procedure described in Example 1 was repeated, except that the temperature was reduced to 80°C, the pressure was increased to 34.5 bar (500 psi), the source of promoter was 30% HBr in acetic acid (2.16 g, 8 meq HBr), and [Rh(CO)₂Br]₂ (0.19 g, 0.8 meq Rh) was used in place of [Rh(CO)₂I]₂. Analysis gave the following results:

| | |
|---|---|
| 3-pentenoic acid (cis and trans) | 24.1 |
| trans-2-pentenoic acid | 0.3 |
| 2-methylbutyric acid | 0.0 |
| valeric acid | 0.0 |
| γ-valerolactone | 0.0 |
| adipic acid | 0.0 |
| 2-methylglutaric acid | 0.0 |
| ethylsuccinic acid | 0.0 |

This Example illustrates that HBr can also be used to give very high selectivity (100%) to unsaturated monocarboxylic acids under somewhat higher temperatures and pressures.

### Example 5. Carbonylation of crotyl alcohol using a bromide promoter at 230°C and 62.1 bar (900 psi).

The procedure described in Example 4 was repeated, except that the temperature was increased to 230°C and the pressure was increased to 62.1 bar (900 psi) (at 230°C). Analysis gave the following results:

| | |
|---|---|
| 3-pentenoic acid (cis and trans) | 2.7 |
| trans-2-pentenoic acid | 1.0 |
| 2-methylbutyric acid | 3.8 |
| valeric acid | 10.1 |
| γ-valerolactone | 15.2 |
| adipic acid | 18.8 |
| 2-methylglutaric acid | 13.8 |
| ethylsuccinic acid | 3.9 |

This Example illustrates the effect of high temperatures.

### Example 6. Carbonylation of 3-buten-2-ol at 200°C and 34.5 bar (500 psi).

The procedure described in Example 1 was repeated, except that the crotyl alcohol was replaced by an equivalent amount of 3-buten-2-ol, the temperature was increased to 200°C and the pressure was increased to 34.5 bar (500 psi). Analysis gave the following results:

| | |
|---|---|
| 3-pentenoic acid (cis and trans) | 1.4 |
| trans-2-pentenoic acid | 0.4 |
| 2-methylbutyric acid | 3.7 |
| valeric acid | 7.4 |
| γ-valerolactone | 4.2 |
| adipic acid | 31.1 |
| 2-methylglutaric acid | 38.5 |
| ethylsuccinic acid | 9.8 |

This Example illustrates that secondary allylic alcohols can be carbonylated as easily as terminal allylic alcohols. It also demonstrates that the site of CO insertion is not necessarily the C-O bond of the allylic substrate.

### Example 7. Carbonylation of 3-buten-2-ol at 80°C and 13.8 bar (200 psi).

The procedure described in Example 6 was repeated, except that the temperature was decreased to 80°C and the pressure was decreased to 13.8 bar (200 psi). Analysis gave the following results:

| | |
|---|---|
| 3-pentenoic acid (cis and trans) | 68.1 |
| trans-2-pentenoic acid | 0.2 |
| 2-methylbutyric acid | 0.4 |
| valeric acid | 0.5 |
| γ-valerolactone | 0.8 |
| adipic acid | 1.2 |
| 2-methylglutaric acid | 11.0 |
| ethylsuccinic acid | 2.0 |

This Example illustrates that a secondary allylic alcohol can be carbonylated under very mild conditions.

### Example 8. Carbonylation of crotyl alcohol in methylene chloride at 140°C.

A 300 mL Hastelloy-C mechanically stirred autoclave was flushed with nitrogen and then with high purity CO. It was then charged with 150 mL of a methylene chloride solution containing [Rh(COD)Cl]₂ (0.37 g, 1.5 mmole), crotyl alcohol (10.8 g, 150 mmole), methanol (0.48 g, 15 mmole), and 5.0 g o-dichlorobenzene (internal standard). The autoclave was closed and pressured with CO pressure to 20.7 bar (300 psi) and then heated to 140°C. The reaction was initiated by injecting into the autoclave 3.33 g of 58% aq HI (15 mmole HI, 78 mmoles of water). The pressure was maintained constant at 29.0 bar (420 psi) by feeding CO from a reservoir at an initial pressure of 81.4 bar (1180 psi). The carbonylation rate was measured by monitoring the reservoir pressure drop.

Uptake of CO occurred rapidly over 15 min and then more slowly over 5 h.

The reaction was allowed to run for a total of about 5 h, after which it was cooled to room temperature. The excess CO was vented and the product was discharged. The autoclave was washed first with 200 mL of methanol at 100°C under autogenous pressure and then with 150 mL methanol at room temperature.

The product and washes from the autoclave were combined, filtered and then the filtrate was diluted to 500 mL with methanol. A sample of this solution was esterified by heating it in a sealed vial at 90°C for 14 h with p-toluenesulfonic acid esterification catalyst and excess methanol. The methyl esters were analyzed and the results are shown below:

| | |
|---|---|
| t-3-pentenoic acid | 4.5% |
| c-3-pentenoic acid | 0.5% |
| 4-pentenoic acid | 0.0% |
| hydroxyvaleric acids | 7.9% |
| mixed butenes (from vapor phase) | 7.4% |
| adipic acid | 27.8% |
| 2-methylglutaric acid | 15.0% |
| ethylsuccinic acid | 1.7% |

No other products were detected in significant amounts, and no tars were formed. This example illustrates the use of a chlorocarbon solvent.

### Example 9. Carbonylation of crotyl alcohol in methylene chloride at 140°C.

The procedure described in Example 10 was repeated using [Rh(COD)Cl]₂ (0.185 g, 0.75 mmole), crotyl alcohol (21.6 g, 300 mmole), and 1.7 g of 57% aq HI (7.5 mmole HI, 39 mmoles of water).

Uptake of CO was rapid and essentially complete in about 30 min. The initial rate corresponds to a half-life of about 18.7 min for the carbonylation of crotyl alcohol. The reservoir pressure drop (10.2 bar (148 psi)) corresponds to about 97 moles of CO per 100 moles of crotyl alcohol charged.

Analysis of the reactor vapor phase showed butadiene formation and decay (initial concentration of 0.075% rising to 0.171% at 54 min and falling to 0.008% at 73 min). 1-Butene and 2-butene were also formed (total 0.74% at 73 min, corresponding to about 5.8 mole% conversion of crotyl alcohol).

The reaction was allowed to run for a total of about 75 min, after which it was cooled to 20°C.

The methyl esters were analyzed and the results are shown below:

| | |
|---|---|
| t-3-pentenoic acid | 53.2% |
| c-3-pentenoic acid | 17.0% |
| 4-pentenoic acid | 0.5% |
| hydroxyvaleric acids | 3.6% |
| mixed butenes (from vapor phase) | 5.8% |
| adipic acid | 5.5% |
| 2-methylglutaric acid | 4.8% |
| ethylsuccinic acid | 0.6% |

No other products were detected in significant amounts, and no tars were formed. This Example illustrates that using a lower water/substrate ratio results in higher ratio of monocarboxylic acid/dicarboxylic acid products.

### Example 10. Carbonylation of crotyl alcohol at ambient temperature.

The procedure described in Example 1 was repeated except that the initial temperature was 25°C and the initial pressure was 6.90 bar (100 psi). At the end of 2 h, the temperature had risen to 38°C although the pressure remained at 6.90 bar (100 psi).

Analysis of the product gave the following results:

| | |
|---|---|
| 3-pentenoic acid (cis and trans) | 26.5% |
| trans 2-pentenoic acid | 0.0% |
| γ-valerolactone | 0.0% |
| valeric acid | 0.0% |
| 2-methylbutyric acid | 0.0% |
| adipic acid | 0.0% |
| 2-methylglutaric acid | 0.0% |
| ethylsuccinic acid | 0.0% |

### Example 11. Carbonylation of crotyl alcohol at 160°C.

The procedure described in Example 1 was repeated except that the acetic acid was replaced with toluene, the initial temperature was 160°C, the initial CO pressure was 20.7 bar (300 psi), the rhodium catalyst was [Rh(COD)Cl]₂ (0.10 g, 0.4 meq Rh) and the aq HI was reduced to 0.3 g (1.35 meq HI). No additional water was added. At the end of 2 h, the temperature was 160°C and the pressure was 30.0 bar (435 psi).

Analysis of the product gave the following results:

| | |
|---|---|
| 3-pentenoic acid (cis and trans) | 64.8% |
| trans 2-pentenoic acid | 1.3% |
| γ-valerolactone | 0.8% |
| valeric acid | 1.0% |
| 2-methylbutyric acid | 0.2% |
| adipic acid | 3.4% |
| 2-methylglutaric acid | 2.5% |
| ethylsuccinic acid | 0.5% |

### Example 12. Crotyl Acetate Carbonylation to 3-Pentenoic Acid in Acetic Acid solvent at 140°C and 20.7 bar (300 psi).

Crotyl acetate was prepared in-situ by heating at reflux under nitrogen a solution of crotyl alcohol (14.4 g; 200 mmoles), acetic anhydride (20.4 g; 200 mmole) and 57% aq HI (3.0 g; 13.5 meq HI) in 212.2 g acetic acid for 2 hours. GC/MS analysis showed that essentially all of the alcohol was converted to a mixture of the acetate isomers under these conditions.

To 50 ml of the above solution (containing 40 mmole crotyl acetate and 2.7 mmole HI) was added an additional 2.04 g (20 mmole) acetic anhydride (to remove water from the aqueous HI) and 0.16 g [Rh(COD)Cl]₂ (0.64 meq Rh). This solution was heated in a glass lined shaker tube at 140°C and 20.7 bar (300 psi) cold CO pressure for 30 minutes as described in Ex. 1.

Analysis of the product showed that the only major products were 3-pentenic acid isomers and acetic anhydride. Analysis of the product after work up in methanol/BF3 gave the following results:

| | |
|---|---|
| Adipic acid | 0.3 |
| 2-Methylglutaric Acid | 0.4 |
| Ethylsuccinic Acid | 0.0 |
| 2-Methylbutyric Acid | 0.0 |
| Valeric Acid | 0.0 |
| 3-Pentenoic acid (cis and trans isomers) | 62.1 |
| Trans-2-Pentenoic Acid | 6.9 |
| γ-Valerolactone | 3.5 |

(When this Example was repeated in the presence of water and for a longer reaction time and/or at higher temperature, 3-pentenoic acid was converted to adipic acid.)

### Example 13. Crotyl Acetate Carbonylation to 3-Pentenoic Acid in Acetic Acid solvent at 80°C and 6.90 bar (100 psi).

When Example 12 was repeated at 80°C and an initial cold pressure of 6.90 bar (100 psi) CO and the product worked up with BF₃/methanol, the following results were obtained:

| | |
|---|---|
| Adipic Acid | 0.0 |
| 2-Methylglutaric Acid | 0.0 |
| Ethylsuccinic Acid | 0.0 |
| 2-Methylbutyric acid | 0.0 |
| Valeric Acid | 0.0 |
| 3-Pentenoic Acid (cis and trans isomers) | 54.9 |
| Trans-2-Pentenoic Acid | 2.5 |
| γ-Valerolactone | 0.0 |

### Example 14. Crotyl Acetate Carbonylation to 3-Pentenoic Acid in Acetic Acid solvent at 40°C and 6.90 bar (100 psi).

Example 12 was repeated except that the catalyst was [Rh(CO)₂Cl]₂, the temperature was reduced to 40°C, the initial cold CO pressure was 6.90 bar (100 psi) and no additional acetic anhydride was added. Product work-up with BF₃/methanol gave the following results:

| | |
|---|---|
| Adipic Acid | 0.0 |
| 2-Methylglutaric Acid | 0.6 |
| Ethylsuccinic Acid | 0.0 |
| 2-Methylbutyric Acid | 0.0 |
| Valeric Acid | 0.0 |
| 3-Pentenoic Acid (cis and trans isomers) | 61.1 |
| Trans-2-Pentenoic Acid | 3.3 |
| γ-Valerolactone | 0.0 |

### Example 15. Carbonylation of crotyl heptanoate at 140°C and 20.7 bar (300 psi).

Crotyl heptanoate was prepare in-situ by heating at reflux under nitrogen a solution of crotyl alcohol (2.9 g, 40 mmole) and 57% aq HI (0.6 g, 2.7 meq) in heptanoic acid (30 mL) for 2 h. Analysis showed that essentially all of the alcohol was converted to a mixture of the heptanoate isomers under these conditions. Identification of the heptanoate isomers was confirmed by analysis.

To the above solution of crotyl heptanoate was added an additional 16 mL of heptanoic acid, water (0.7 g, 40 mmole), and RhCl₃.3H₂O (0.21 g, 0.8 meq Rh). This solution was carbonylated in a glass-lined shaker tube at 140°C and 20.7 bar (300 psi) cold CO pressure for 2 h as described in Example 1.

Analysis of the product gave the following results:

| | |
|---|---|
| 3-Pentenoic Acid (cis and trans) | 21.7% |
| trans-2-pentenoic acid | 1.5% |
| γ-valerolactone | 2.5% |
| valeric acid | 1.9% |
| 2-methylbutyric acid | 0.0% |
| adipic acid | 12.4% |
| 2-methylglutaric acid | 18.3% |
| ethylsuccinic acid | 4.3% |

## Claims

1. A process for the carbonylation of an allylic compound having the formula : 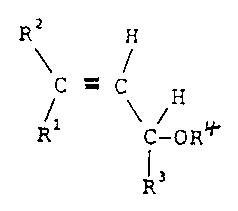 where
one of the groups R¹, R² and R³ is methyl and the other two groups are H; and
R⁴ is selected from the group consisting of H and 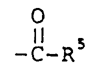 wherein R⁵ is selected from the group consisting of C₁ to C₁₀ alkyl, to form 3-pentenoic acid or a carboxylic acid anhydride derivative thereof, which comprises reacting said allylic compound and carbon monoxide with a rhodium catalyst and a promoter selected from the class consisting of hydrogen bromide and hydrogen iodide at a temperature in the range of 10°C to 250°C and a carbon monoxide partial pressure in the range of 1.7 to 206.9 bar gauge (25 to 3000 psig), wherein the concentration of rhodium is in the range of 0.005% to 0.50% by weight of the total weight of the reaction mixture, and the molar ratio of promoter to rhodium is between 1:1 and 10:1.

2. A process according to claim 1, wherein the concentration of rhodium is in the range of 0.01% to 0.20% by weight of the total weight of the reaction mixture.

3. A process according to claim 2, wherein the concentration of rhodium is in the range of 0.02% to 0.10% by weight of the total weight of the reaction mixture.

4. A process according to claim 1, wherein the reaction is conducted in the presence of at least one solvent chosen from the group of aliphatic carboxylic acids, aromatic carboxylic acids, aromatic hydrocarbons, and saturated halocarbons.

5. A process according to claim 1, wherein the partial pressure of carbon monoxide is 6.90 to 69.0 bar gauge (100 to 1000 psig).

6. A process according to claim 1, wherein the promoter is hydrogen iodide.

7. A process according to claim 6, wherein the ratio of the promoter to rhodium is between 2:1 and 6:1.

8. A process according to claim 1, wherein the allylic compound is chosen from the group of 2-buten-1-ol and 3-buten-2-ol, and the product is 3-pentenoic acid.

9. A process according to claim 1, wherein the allylic compound is an allylic butenol ester of a carboxylic acid, and the product is a carboxylic acid anhydride derivative of 3-pentenoic acid.

10. A process according to claim 1, wherein said allylic butenol compound is crotyl acetate, the carbon monoxide partial pressure is in the range of 6.90 to 69.0 bar gauge (100-1000 psig), the concentration of rhodium is in the range of 0.01-0.20% based on the total weight of the reaction mixture, and the molar ratio of promoter to rhodium is between 2:1 and 6:1 and wherein water is present.

## Patentansprüche

1. Ein Verfahren für die Carbonylierung einer Allylverbindung mit der Formel: 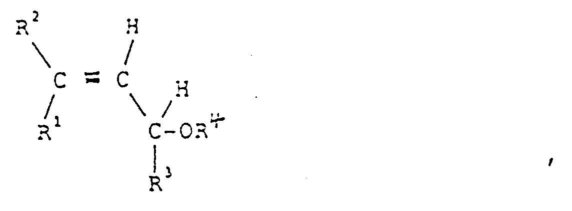 worin
eine der Gruppen R¹, R² und R³ Methyl ist, und die anderen zwei Gruppen H sind, und
R⁴ ausgewählt ist aus der Gruppe, bestehend aus H und 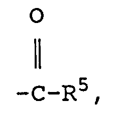 worin R⁵ ausgewählt ist aus der Gruppe, bestehend aus C₁- bis C₁₀-Alkyl, um 3-Pentensäure oder ein Carbonsäureanhydrid-Derivat davon zu bilden, das Umsetzen der Allylverbindung und Kohlenmonoxid mit einem Rhodiumkatalysator und einem Promotor, ausgewählt aus der Klasse, bestehend aus Bromwasserstoff und Iodwasserstoff, bei einer Temperatur in dem Bereich von 10°C bis 250°C und einem Kohlenmonoxid-Partialdruck in dem Bereich von 1,7 bis 206,9 bar Überdruck (25 bis 3000 psig) umfaßt, worin die Rhodiumkonzentration in dem Bereich von 0,005 bis 0,50 Gewichtsprozent des Gesamtgewichts der Reaktionsmischung ist, und das Molverhältnis von Promotor zu Rhodium zwischen 1:1 und 10:1 ist.

2. Ein Verfahren gemäß Anspruch 1, worin die Rhodiumkonzentration in dem Bereich von 0,01 bis 0,20 Gewichtsprozent des Gesamtgewichts der Reaktionsmischung ist.

3. Ein Verfahren gemäß Anspruch 2, worin die Rhodiumkonzentration in dem Bereich von 0,02 bis 0,10 Gewichtsprozent des Gesamtgewichts der Reaktionsmischung ist.

4. Ein Verfahren gemäß Anspruch 1, worin die Reaktion in Gegenwart von zumindest einem Lösungsmittel, ausgewählt aus der Gruppe aliphatischer Carbonsäuren, aromatischer Carbonsäuren, aromatischer Kohlenwasserstoffe und gesättigter Halogenkohlenwasserstoffe, ausgeführt wird.

5. Ein Verfahren gemäß Anspruch 1, worin der Kohlenmonoxid-Partialdruck 6,90 bis 69,0 bar Überdruck (100 bis 1000 psig) ist.

6. Ein Verfahren gemäß Anspruch 1, worin der Promotor Iodwasserstoff ist.

7. Ein Verfahren gemäß Anspruch 6, worin das Verhältnis des Promotors zu Rhodium zwischen 2:1 und 6:1 ist.

8. Ein Verfahren gemäß Anspruch 1, worin die Allylverbindung ausgewählt ist aus der Gruppe aus 2-Buten-1-ol und 3-Buten-2-ol, und das Produkt 3-Pentensäure ist.

9. Ein Verfahren gemaß Anspruch 1, worin die Allylverbindung ein Allylbutenolester einer Carbonsäure ist, und das Produkt ein Carbonsäureanhydrid-Derivat von 3-Pentensäure ist.

10. Ein Verfahren gemäß Anspruch 1, worin die Allylbutenolverbindung Crotylacetat ist, der Kohlenmonoxid-Partialdruck in dem Bereich von 6,90 bis 69,0 bar Überdruck (100 bis 1000 psig) ist, die Rhodiumkonzentration in dem Bereich von 0,01 bis 0,20 % bezogen auf das Gesamtgewicht der Reaktionsmischung ist, und das Molverhältnis von Promotor zu Rhodium zwischen 2:1 und 6:1 ist, und worin Wasser anwesend ist.

## Revendications

1. Un procédé de carbonylation d'un dérivé allylique répondant à la formule: 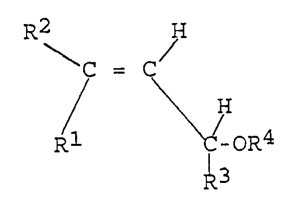 dans laquelle:
- l'un des groupes R¹, R² et R³ est un groupe méthyle et les deux autres groupes représentent H; et
- R⁴ est choisi dans le groupe constitué par H et 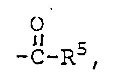 R⁵ étant choisi dans le groupe constitué par un radical alkyle en C₁ à C₁₀,
pour donner de l'acide 3-penténoïque ou un de ses dérivés anhydride d'acide carboxylique, qui comprend la réaction dudit dérivé allylique avec du monoxyde de carbone en présence d'un catalyseur à base de rhodium et d'un promoteur choisi dans la classe constituée par l'acide bromhydrique et l'acide iodhydrique, à une température comprise entre 10 et 250°C et sous une pression partielle de monoxyde de carbone comprise entre 1,7 et 206,9 bars relatifs (25 à 3000 psig), la concentration en rhodium étant comprise entre 0,005% et 0,50%. par rapport au poids total du mélange réactionnel et le rapport molaire du promoteur au rhodium étant compris entre 1/1 et 10/1.

2. Un procédé selon la revendication 1, dans lequel la concentration en rhodium est comprise entre 0,01 et 0,20% par rapport au poids total du mélange réactionnel.

3. Un procédé selon la revendication 2, dans lequel la concentration en rhodium est comprise entre 0,02 et 0,10% par rapport au poids total du mélange réactionnel.

4. Un procédé selon la revendication 1, dans lequel on effectue la réaction en présence d'au moins un solvant choisi dans le groupe constitué par des acides carboxyliques aliphatiques, acides carboxyliques aromatiques, hydrocarbures aromatiques et hydrocarbures halogénés saturés.

5. Un procédé selon la revendication 1, dans lequel la pression partielle de monoxyde de carbone est comprise entre 6,90 et 69,0 bars relatifs (100 et 1000 psig).

6. Un procédé selon la revendication 1, dans lequel le promoteur est l'acide iodhydrique.

7. Un procédé selon la revendication 6, dans lequel le rapport du promoteur au rhodium est compris entre 2/1 et 6/1.

8. Un procédé selon la revendication 1, dans lequel le dérivé allylique est choisi dans le groupe constitué par le 2-buten-1-ol et le 3-buten-2-ol et le produit est l'acide 3-penténoïque.

9. Un procédé selon la revendication 1, dans lequel le dérivé allylique est un ester de buténol allylique d'un acide carboxylique et le produit est un anhydride d'acide carboxylique dérivé de l'acide 3-penténoïque.

10. Un procédé selon la revendication 1, dans lequel ledit dérivé de buténol allylique est l'acétate de crotyle, la pression partielle de monoxyde de carbone est comprise entre 6,90 et 69,0 bars relatifs (100 et 1000 psig), la concentration du rhodium est comprise entre 0,01 et 0,20% par rapport au poids total du mélange réactionnel, le rapport molaire du promoteur au rhodium est compris entre 2/1 et 6/1 et dans lequel de l'eau est présente.
